# EUROPEAN PATENT APPLICATION

(11) **EP 4 501 594 A1**
(43) Date of publication of application: **05.02.2025**
(21) Application number: 23791814.9
(22) Date of filing: 17.04.2023
(51) Int. Cl.: B29C 65/08, A61F 13/15, A61F 13/496, B23K 20/10

(54) **ULTRASOUND WELDING APPARATUS AND METHOD**

(30) Priority: 21.04.2022 JP 2022069979
(71) Applicant: Zuiko Corporation, Ibaraki-shi Osaka 5670082 (JP)
(72) Inventor: IKEDA, Natsuki, Ibaraki-shi, Osaka 567-0082 (JP)
(74) Representative: Hasegawa, Kan
(86) International application number: PCT/JP2023/015283
(87) International publication number: WO 2023/204166

(57) **Abstract**

Including a process of applying ultrasonic energy to a workpiece; a process of reciprocally moving an anvil between an overrun region and a non-welding region; a process of guiding the anvil such that the anvil reaches a first level away from a surface level of a horn so that the anvil does not abut against the horn at ends portions far from a welding region and moving the anvil to a second level where the anvil touches the horn in the welding region; and a process of performing control such that in the overrun region the anvil becomes displaced from the first level to the second level and the anvil abuts against the horn not via the workpiece in a non-driven state in which the horn is not being caused to generate ultrasonic energy.

## Description

### TECHNICAL FIELD

The present invention relates to an ultrasonic welding apparatus and method.

### BACKGROUND ART

Apparatuses are known which have an ultrasonic horn that rotates together with a drum and an anvil that cooperates with the horn, and as the drum rotates, the anvil reciprocates in the axial direction of the drum to perform a welding process on a workpiece, having superposed webs, sandwiched and held between the horn and the anvil (Patent Document 1).

### CITATION LIST

### Patent Literature

Patent Document 1: Japanese Patent No. 6228232 (Abstract)

### SUMMARY OF INVENTION

In this related art, the welding process is performed during both outbound and inbound movements of the anvil, but misalignment may occur between the welding marks on the outbound path and the welding marks on the inbound path due, for example, to misalignment of the webs between the outbound and inbound movements of the anvil, so that there have been the problems that the weld strength becomes unstable and the outward appearance is diminished.

It is proposed that control be performed so that the horn be vibrated via an ultrasonic generator to apply ultrasonic energy to the workpiece on either one of the outbound path or the inbound path of the anvil and ultrasonic energy be not applied to the workpiece on the other of the outbound path or the inbound path.

According to the example of this proposal, welding of the workpiece is performed only on one of the outbound path or the inbound path. For that reason, misalignment of the welding marks does not occur, so the welding strength does not become unstable and the outward appearance is not diminished.

Here, in the region of the moving range of the anvil where the workpiece is present between the anvil and the horn, it is necessary to bring the anvil close to the horn to weld the workpiece. Whereas, in the regions of the moving range of the anvil where the workpiece is not present between the anvil and the horn, it is necessary to keep the anvil away from the horn to suppress wear and deterioration of the anvil and the horn.

Meanwhile, by controlling the sonic output from the horn, a stable welding state is obtained and deterioration of the horn is reduced. For that reason, a threshold value is set for the upper limit of the sonic output, and when this is exceeded, an error output is generated.

However, when the anvil approaches the horn from a first level where the anvil is away from the horn to a second level where the horn abuts against the anvil, shocks are produced in the horn when the anvil impacts on the horn.

Such shocks are a reason the sonic output exceeds the upper limit value and can cause an error output to be displayed and stoppage of the apparatus, and/or the horn to deteriorate.

Consequently, it is an object of the present invention to provide an ultrasonic welding apparatus and method that can reduce shocks produced in the horn to prevent errors and inhibit deterioration of the horn.

The apparatus of the present invention is an ultrasonic welding apparatus 1 that forms a weld portion S in a workpiece W such that a welding region α in which the workpiece W is welded extends in a first direction L1 and a non-welding region Δ that extends on one side of the welding region α and in which it is not necessary to weld the workpiece W is consecutive with the welding region α in the first direction L1, the ultrasonic welding apparatus 1 comprising:
a horn 6 and an anvil 10;
an ultrasonic generator 16 configured to cause the horn 6 to vibrate to apply ultrasonic energy to the workpiece W sandwiched between the anvil 10 and the horn 6;
a moving mechanism configured to move the anvil 10 in the first direction L1 such that the horn 6 abuts against the anvil 10 via the workpiece W in the welding region α and the non-welding region Δ and reciprocally move the anvil 10 between an overrun region β, where the anvil 10 exceeds an other side of the welding region α, and the non-welding region Δ;
a displacement mechanism configured to guide the anvil 10 such that the anvil 10 reaches a first level away from a surface level of the horn 6 so that the anvil 10 does not abut against the horn 6 at end portions (stroke ends of the anvil) of the non-welding region Δ and the overrun region β far from the welding region α and allow the anvil 10 to move to a second level where the anvil 10 touches the horn 6 in the welding region α; and
a control device 500 that performs control such that in the overrun region β the anvil 10 becomes displaced from the first level to the second level and the anvil 10 abuts against the horn 6 not via the workpiece W in a non-driven state (including a weakly driven state) in which the horn 6 is not being caused to generate ultrasonic energy.

The method of the present invention is an ultrasonic welding method that forms a weld portion S in a workpiece W such that a welding region α in which the workpiece W is welded extends in a first direction L1 and a non-welding region Δ that extends on one side of the welding region α and in which it is not necessary to weld the workpiece W is consecutive with the welding region α in the first direction L1, the ultrasonic welding method comprising:
causing a horn 6 to vibrate to apply ultrasonic energy to the workpiece W sandwiched between an anvil 10 and the horn 6;
moving the anvil 10 in the first direction L1 such that the horn 6 abuts against the anvil 10 via the workpiece W in the welding region α and the non-welding region Δ and reciprocally moving the anvil 10 between an overrun region β, where the anvil 10 exceeds the other side of the welding region α, and the non-welding region Δ;
guiding the anvil 10 such that the anvil 10 reaches a first level away from a surface level of the horn 6 so that the anvil 10 does not abut against the horn 6 at end portions (stroke ends of the anvil) of the non-welding region Δ and the overrun region β far from the welding region α and causing the anvil 10 to move to a second level where the anvil 10 touches the horn 6 in the welding region α; and
performing control such that in the overrun region β the anvil 10 becomes displaced from the first level to the second level and the anvil 10 abuts against the horn 6 not via the workpiece W in a non-driven state (including a weakly driven state) in which the horn 6 is not being caused to generate ultrasonic energy.

In the present invention, "welding a workpiece" may mean welding plural webs that have been superposed on top of each other or performing what is called side sealing where one web laminate that has been folded in two is welded.

### BRIEF DESCRIPTION OF DRAWINGS

FIG. 1 is plan views and a cross-sectional view for describing an outline of processes in a method of manufacturing a disposable diaper pertaining to the present invention.
FIG. 2 is a front view of main parts of an ultrasonic welding apparatus pertaining to the present invention.
FIG. 3 is a cross-sectional view taken along line III-III of FIG. 2.
FIG. 4 is a cross-sectional view taken along line IV-IV of FIG. 3.
FIG. 5 is a view of a sheet retention drum of FIG. 3 as viewed from the outer side thereof.
FIG. 6 is a side view showing a horn, an anvil, and a lift cam.
FIG. 7 is a cross-sectional view taken along line VII-VII of FIG. 6.
FIG. 8 is side views showing the horn, the anvil, and the lift cam.
FIG. 9 is a schematic perspective view showing the operation of the anvil on an outbound path.
FIG. 10 is a schematic perspective view showing the operation of the anvil on an inbound path.
FIG. 11 are characteristic diagrams showing the relationship between the movement of the anvil and the output of the horn.

### DESCRIPTION OF EMBODIMENTS

The present invention will become more clearly understood from the following description of preferred embodiments taken in conjunction with the accompanying drawings. However, the embodiments and the drawings are merely for the purposes of illustration and explanation and should not be utilized to define the scope of the present invention. The scope of the present invention shall be defined only by the claims. In the accompanying drawings, like parts numbers in multiple views refer to the same or corresponding parts.

Prior to describing an apparatus pertaining to an example of the present invention, the structure of and a method of manufacturing a disposable diaper 20, which is an example of a wearable article manufacturing by the same apparatus, will be described below.

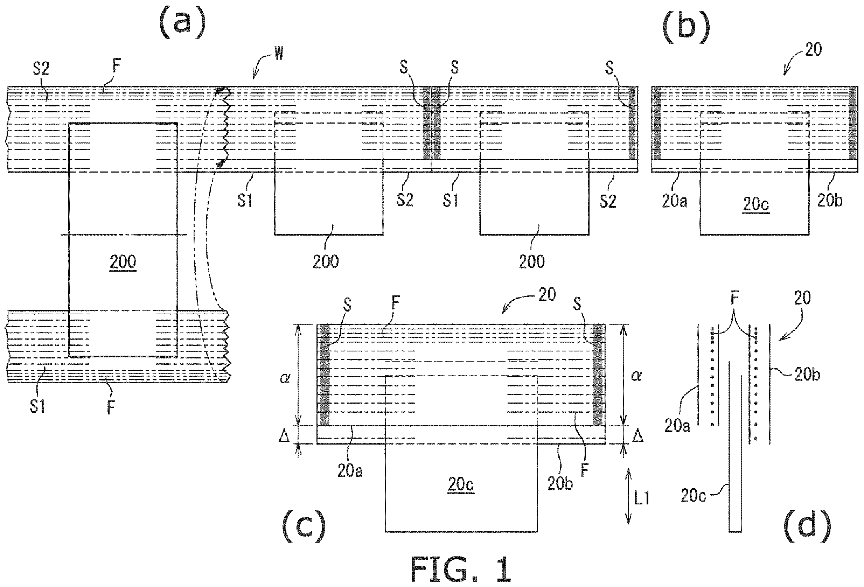

Referring to FIG. 1, the disposable diaper 20 shown in (c) and (d) includes, when it is put on, a front abdominal portion 20a disposed on the abdomen of the wearer, a back portion 20b disposed on the buttocks of the wearer, and a crotch portion 20c that extends from the front abdominal portion 20a between both legs of the wearer to the back portion 20b.

Both side edge portions of the front abdominal portion 20a and both side edge portions of the back portion 20b are welded to each other by two weld portions S such that the front abdominal portion 20a and the back portion 20b are interconnected in a loop.

A method of manufacturing the disposable diaper 20 will now be described below.

In FIG. 1(a), a workpiece W extending in a lengthwise direction is conveyed along its lengthwise direction. The workpiece W is processed into the disposable diaper 20. Hereinafter, the flow direction of the workpiece W will be described as a transverse direction, and the direction orthogonal to the transverse direction in FIG. 1 will be described as a vertical direction.

The workpiece W includes a first stretchable sheet S1 that becomes the front abdominal portion 20a, a second stretchable sheet S2 that becomes the back portion 20b, and an absorber 200 that configures the crotch portion 20c. Each sheet S1, S2 has a skin-side inner sheet that faces the body surface side of the wearer when worn, a non-skin-side outer sheet that faces away from the wearer when worn, and elastic members F that are sandwiched between these.

The inner sheet is configured by a nonwoven fabric sheet and/or a mesh sheet that is permeable to liquid. The outer sheet is configured by the same material as the inner sheet or a polyethylene film, a polypropylene film, or a nonwoven fabric that is water-repellent and breathable.

The elastic members F are configured using sheets or threads including polyurethane, natural rubber, or a thermoplastic resin.

The workpiece W is disposed with the crotch portion 20c bridging the front abdominal portion 20a and the back portion 20b. The workpiece W is folded in two in the crotch portion 20c while it is being conveyed.

The absorber 200 includes a permeable sheet that is permeable to liquid, a water-repellent sheet that is water-repellent and breathable, and an absorbent core that is sandwiched between the permeable sheet and the water-repellent sheet.

The permeable sheet is configured by a nonwoven fabric sheet and/or a mesh sheet that is permeable to liquid. The water-repellent sheet is configured by a polyethylene film, a polypropylene film, or a nonwoven fabric that is water-repellent and breathable.

The absorbent core is molded by laminating pulverized pulp or a mixture of pulverized pulp and a super absorbent polymer.

By folding in two in the vertical direction the workpiece W (continuous body) on which the absorber 200 has been placed, the part of the workpiece W that corresponds to the front abdominal portion 20a and the part that corresponds to the back portion 20b are superposed on each other. At this time, the lower edge portion of the back portion 20b protrudes downward from the front abdominal portion 20a. The part that protrudes becomes a non-welding region Δ that is not welded later.

That is, the workpiece W of FIG. 1 includes the front abdominal portion 20a and the back portion 20b, and the back portion 20b covers the front abdominal portion 20a and has the non-welding region Δ that protrudes on one side in a first direction L1 from the front abdominal portion 20a.

In a next welding process, in the workpiece W folded in two, a part that corresponds to a side edge portion of the front abdominal portion 20a and a part that corresponds to a side edge portion of the back portion 20b are ultrasonically welded.

Specifically, in the welding process, weld portions S are formed in the workpiece W by simultaneously ultrasonically welding two places in the workpiece W, leaving an interval across a preset cutting range as a range in which to cut the workpiece W in a cutting process described later.

It will be noted that the weld portions S are formed in a welding region α in the vertical direction in the part corresponding to the side edge portion of the front abdominal portion 20a and the part corresponding to the side edge portion of the back portion 20b.

In a next cutting process, the workpiece W is cut along a virtual cutting line extending in the vertical direction between the two weld portions S that were formed in the welding process. Because of this, the workpiece W (continuous body) is cut into the disposable diaper 20.

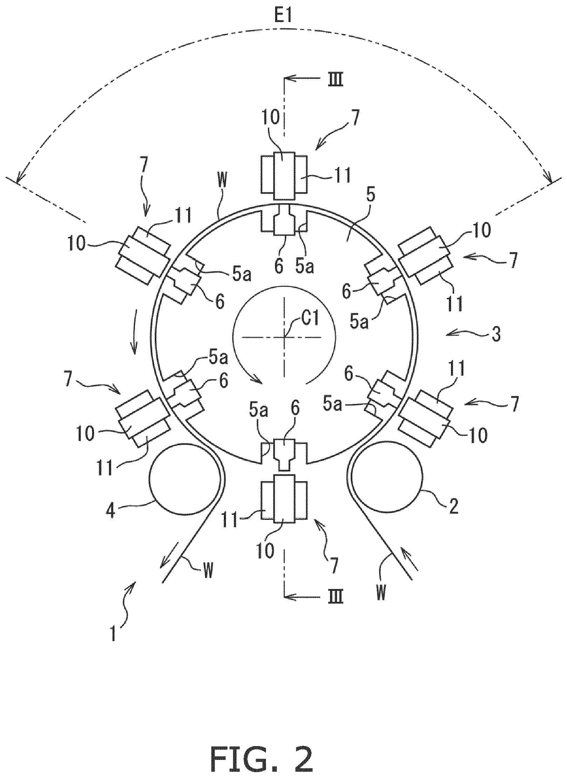

An ultrasonic welding apparatus 1 pertaining to an embodiment that performs the welding process will now be described below with reference to FIG. 2.

The ultrasonic welding apparatus 1 includes an infeed roller 2 that infeeds the workpiece W that has been folded in two in the fold-in-two process, a welding drum 3 that welds the workpiece W that has been infed by the infeed roller 2, and an outfeed roller 4 that outfeeds the workpiece W that has been welded by the welding drum 3.

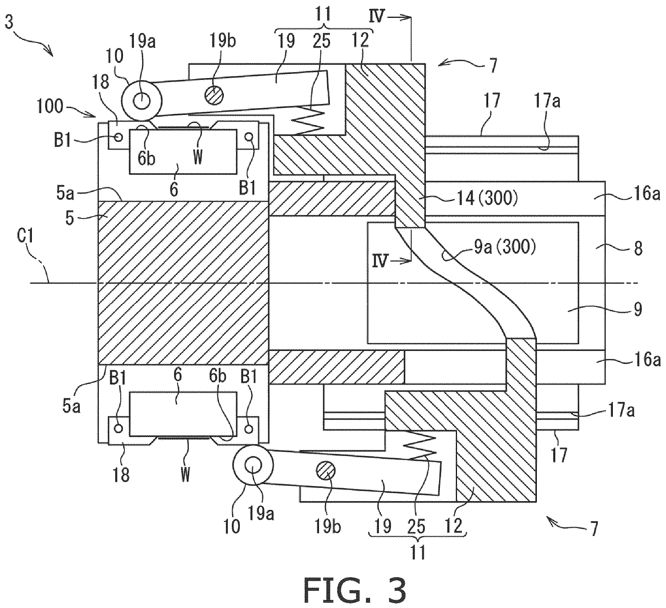

The welding drum 3 includes a sheet retention drum 5 that retains the workpiece W infed by the infeed roller 2, six horns (ultrasonic horns) 6 provided in the sheet retention drum 5, six anvil units 7 that ultrasonically weld the workpiece W between themselves and the ultrasonic horns 6, tubular anvil retention drums 8 (see FIG. 3) that retain the anvil units 7, cam drums 9 (see FIG. 3) that are provided inside the anvil retention drums 8, and six lift cams 18 (see FIG. 3) that are secured to the sheet retention drum 5 adjacent to each ultrasonic horn 6.

The welding drum 3 conveys the workpiece W along its outer peripheral surface while rotating and in which the horns 6 and anvils 10 being disposed oppose to each other inward and outward in its radial direction.

Referring to FIG. 2 and FIG. 3, the sheet retention drum 5 can rotate about a rotational center C1 with the workpiece W retained on its outer peripheral surface. Furthermore, in the sheet retention drum 5, six recessed grooves 5a are formed at equal intervals about the rotational center C1. Each recessed groove 5a opens outward of the sheet retention drum 5 and extends along the rotational center C1.

Each ultrasonic horn 6 applies ultrasonic vibrations to the workpiece W retained by the sheet retention drum 5. Since the ultrasonic horns 6 have the same configuration as one another, just one ultrasonic horn 6 will be described and description of the other ultrasonic horns 6 will be omitted.

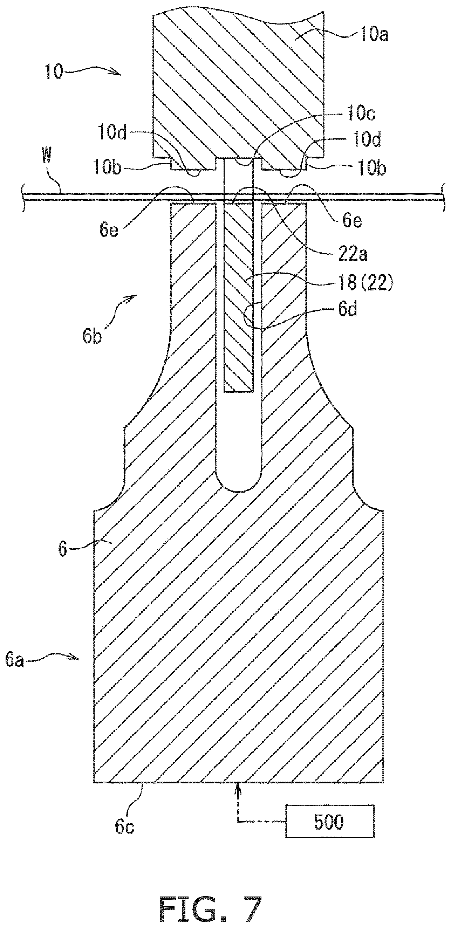

Referring to FIG. 7, the ultrasonic horn 6 includes an input-side end portion 6a having an input surface 6c to which ultrasonic vibrations are input and an output-side end portion 6b having a pair of output surfaces 6e that are bifurcated in a direction orthogonal to the rotational center C1 by a slit 6d extending along the rotational center C1 of the sheet retention drum 5 (see FIG. 2) and output ultrasonic vibrations on both sides of the slit 6d.

The width dimension of the slit 6d is a dimension corresponding to the interval between the two weld portions S that are simultaneously welded in the welding process (see FIG. 1).

The distance between the input surface 6c and each output surface 6e is set to a distance corresponding to a half-wavelength (1/2λ) of the ultrasonic vibrations input to the input surface 6c. The slit 6d is formed in a range from a position corresponding to a node (not shown in the drawings) of the ultrasonic vibrations input to the input surface 6c to the end surfaces (the output surfaces 6e) of the output-side end portion 6b.

It will be noted that the ultrasonic vibrations input to the ultrasonic horn 6 are longitudinal waves in a direction orthogonal to the output surfaces 6e.

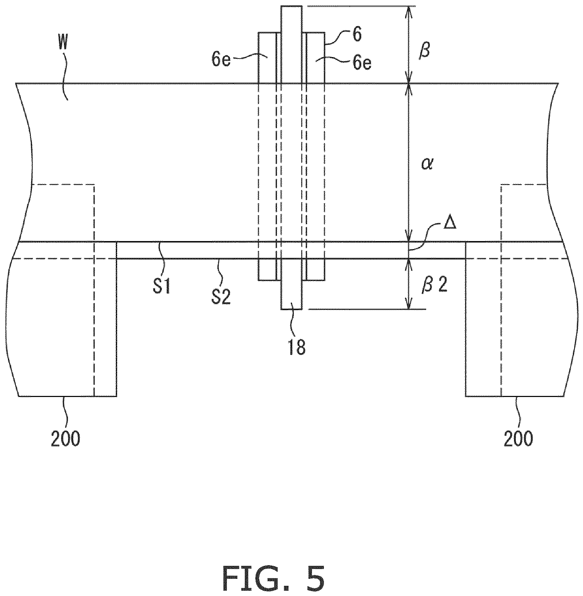

Furthermore, the ultrasonic horn 6 is, as shown in FIG. 2 and FIG. 5, provided inside the recessed groove 5a such that each output surface 6e contacts from inside the workpiece W retained by the sheet retention drum 5.

Here, the length of each output surface 6e is, as shown in FIG. 5, longer than the welding region α set in the workpiece W, and both lengthwise direction end portions of each output surface 6e are disposed so as to project on both sides from the welding region α.

The anvil units 7 of FIG. 3 are provided in the same positions as the ultrasonic horns 6 about the rotational center C1. Since the anvil units 7 have the same configuration as one another, the configuration of one anvil unit 7 will be described and description of the other anvil units 7 will be omitted.

The anvil unit 7 includes an anvil (roller) 10 for welding the workpiece W between itself and the ultrasonic horn 6 and an anvil retention member 11 that retains the anvil roller 10 such that the anvil roller 10 can move relative to the sheet retention drum 5 along the rotational center C 1.

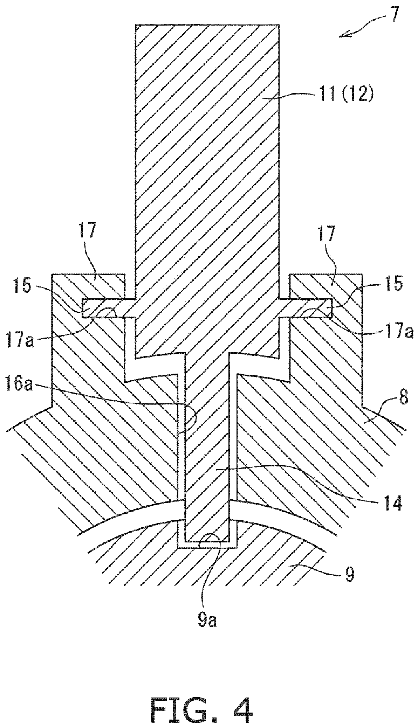

As shown in FIG. 3 and FIG. 4, the retention member 11 includes a retention member body 12 that is attached to the anvil retention drum 8 so as to be movable along the rotational center C1, a retention lever 19 that is attached to the retention member body 12 so as to be pivotable about a pivot shaft 19b of FIG. 3 and retains the anvil roller 10 such that the anvil roller 10 is rotatable about a rotating shaft 19a, and an energizing member 25 that energizes the retention lever 19 in a direction in which the anvil (roller) 10 moves toward the ultrasonic horn 6.

In FIG. 3, the rotating shaft 19a and the pivot shaft 19b are shafts that extend in a direction orthogonal to a plane including the rotational center C1 and the anvil unit 7 (a direction orthogonal to the page surface of FIG. 3). Furthermore, the rotating shaft 19a is provided on the distal end portion of the retention lever 19, and the pivot shaft 19b is provided in an intermediate portion of the retention lever 19.

For that reason, the anvil roller 10 can rollingly contact the workpiece W in accordance with the movement of the retention member 11 along the rotational center C1 and can move toward and away from the workpiece W (the ultrasonic horn 6) in the radial direction of the sheet retention drum 5 in accordance with the pivoting of the retention lever 19.

The energizing member 25 energizes the anvil roller 10 in a direction in which the anvil roller 10 moves toward the ultrasonic horn 6 by energizing the base end portion of the retention lever 19 in a direction away from the rotational center C1 relative to the retention member body 12.

The energizing member 25, the retention lever 19, and the pivot shaft 19b energize the anvil roller 10 toward the ultrasonic horn 6 so that the output surfaces 6e of the ultrasonic horn 6 and welding surfaces 10d of the anvil roller 10 described later move toward to each other in directions normal to these surfaces, and configure part of a displacement mechanism 100.

The retention member body 12 is provided with a cam protrusion 14 that extends toward the rotational center C1 and a pair of engagement protrusions 15 of FIG. 4 that project oppositely of each other in directions (right and left directions in FIG. 4) orthogonal to the cam protrusion 14 and the rotational center C1 and extend along the rotational center C1.

In FIG. 4, the retention member body 12 is provided between a pair of rails 17 erected on the outer peripheral surface of the anvil retention drum 8. In each rail 17 is formed an engagement groove 17a that opens toward the other rail 17 and extends along the rotational center C1 (FIG. 3). The engagement protrusions 15 of the retention member body 12 are engaged with the engagement grooves 17a so as to be movable along the rotational center C1 relative to the anvil retention drum 8.

In FIG. 3, the tubular anvil retention drum 8 of FIG. 3 is provided with a slit 16a that runs through its peripheral wall and extends along the rotational center C1. The cam protrusion 14 of the retention member body 12 is inserted inside the anvil retention drum 8 through the slit 16a.

Next, an example of a moving mechanism 300 will be described. Inside the anvil retention drum 8 is provided a cam drum 9, and in the outer peripheral surface of the cam drum 9 is formed a cam groove 9a. The distal end portion of the cam protrusion 14 is inserted inside the cam groove 9a. The cam groove 9a guides the cam protrusion 14 such that the anvil unit 7 moves along the rotational center C1 as the anvil retention drum 8 rotates relative to the cam drum 9.

Here, the sheet retention drum 5 and the anvil retention drum 8 are secured to each other, and both rotate integrally about the rotational center C1. At the same time, the rotational position of the cam drum 9 is fixed regardless of the rotation of the sheet retention drum 5 and the anvil retention drum 8. Consequently, the retention member body 12 moves along the rotational center C1 in accordance with the rotation of the sheet retention drum 5 and the anvil retention drum 8 about the rotational center C1.

Specifically, the lowest positioned anvil unit 7 in FIG. 2 and FIG. 3 is disposed in a position offset from the workpiece W retained by the sheet retention drum 5 as viewed in a plan view. In this state, as the sheet retention drum 5 rotates counterclockwise in FIG. 2, the anvil unit 7 moves in a direction toward the workpiece W along the rotational center C1.

In the process of the anvil unit 7 rotating to the uppermost position in FIG. 2 and FIG. 3, the anvil roller 10 crosses over the workpiece W, and in the uppermost positioned anvil unit 7 in FIG. 2 and FIG. 3, the anvil roller 10 is disposed in a position offset from the workpiece W retained by the sheet retention drum 5 as viewed in a plan view. When the sheet retention drum 5 further rotates counterclockwise from this state, the anvil roller 10 again crosses over the workpiece W and returns to the position of the anvil roller 10 in the lowest position in FIG. 2 and FIG. 3.

In other words, in the ultrasonic welding apparatus 1, in range E1 of FIG. 2 the anvil roller 10 reciprocally moves over the workpiece W in the welding region α of FIG. 1, and during this reciprocating motion the workpiece W is welded. More specifically, the anvil roller 10 positioned in the range outside the range E1 in FIG. 2 is positioned in first and second overrun regions β, β2 of FIG. 5 away from the workpiece W as viewed in a plan view, and when the anvil roller 10 enters the range E1, the anvil roller 10 becomes positioned in the welding region α and the non-welding region Δ coinciding with the workpiece W as viewed in a plan view and sequentially moves through the second overrun region β2, which is away from the workpiece W on the opposite side of the first overrun region β as viewed in a plan view, and the welding region α and non-welding region Δ.

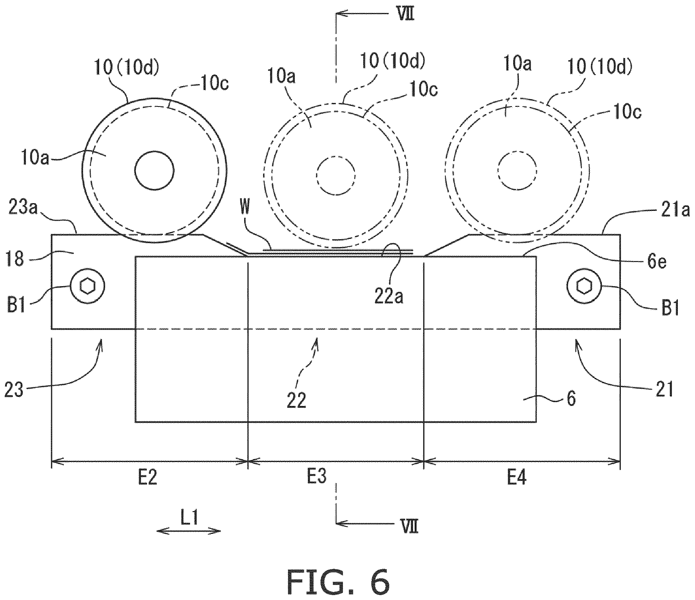

The lift cam 18 of FIG. 6 for keeping the anvil roller 10 positioned in the overrun regions β, β2 away from the workpiece W will now be described below with reference to FIG. 5 to FIG. 7.

The lift cam 18 of FIG. 7 is a tabular member that has a width dimension smaller than the width dimension of the slit 6d in the ultrasonic horn 6 and is provided in the width direction range of the slit 6d. Furthermore, the width dimension of the lift cam 18 is smaller than an interval between a pair of projecting portions 10b of the anvil roller 10. Here, the projecting portions 10b project from, and all the way around, an outer peripheral surface of a disk-shaped roller body 10a and have welding surfaces 10d that weld the workpiece W between themselves and the output surfaces 6e of the ultrasonic horn 6. The part of the outer peripheral surface of the roller body 10a between both projecting portions 10b configures a later-described supported surface 10c that can rollingly contact the lift cam 18.

The lift cam 18 of FIG. 5 has a length dimension larger than the length dimension of the ultrasonic horn 6 and is disposed along the rotational center C1. For example, both lengthwise direction end portions of the lift cam 18 are disposed outside the ultrasonic horn 6 and are secured to the sheet retention drum 5 (FIG. 3) by a pair of bolts B1 in FIG. 6.

The lift cam 18 of FIG. 6 includes a base end side guide portion 21 disposed in a non-contact range E4, a central portion 22 disposed in a contact range E3, and a distal end side guide portion 23 disposed in a non-contact range E2.

The central portion 22 has a non-guide surface 22a disposed in the same plane as the output surfaces 6e of the ultrasonic horn 6 or in a position on the rotational center C1 side of the output surfaces 6e.

The base end side guide portion 21 has a base end side guide surface 21a including a sloping surface, which slopes outward in the radial direction of the sheet retention drum 5 from the non-guide surface 22a toward a direction away from the central portion 22, and a flat surface, which extends in a direction parallel to the non-guide surface 22a (the output surfaces 6e) from the sloping surface toward a direction away from the central portion 22.

Likewise, the distal end side guide portion 23 has a distal end side guide surface 23a including a sloping surface, which slopes outward in the radial direction of the sheet retention drum 5 from the non-guide surface 22a toward a direction away from the central portion 22, and a flat surface, which extends in a direction parallel to the non-guide surface 22a from the sloping surface toward a direction away from the central portion 22.

Against the guide surfaces 21a, 23a, the supported surface 10c of the anvil roller 10 positioned in the first and second overrun regions β, β2 (FIG. 5) is pressed by the energizing force of an energizing mechanism (the energizing member 25, the retention lever 19, and the pivot shaft 19b). Furthermore, the lift cam 18 is secured to the sheet retention drum 5 in a state in which both guide surfaces 21a, 23a are disposed in the width direction range of the slit 16a (FIG. 4).

In FIG. 6, both guide surfaces 21a, 23a keep the anvil roller 10 that is positioned in the non-contact ranges E2, E4 away from the workpiece W (the ultrasonic horn 6) by rollingly contacting the supported surface 10c of the anvil roller 10 as the anvil roller 10 moves from the contact range E3 to the non-contact ranges E2, E4. Here, the supported surface 10c is the outer peripheral surface (outer surface) of the roller body 10a positioned between the projecting portions 10b of the anvil roller 10.

In other words, the end portion of the base end side guide portion 21 including the base end side guide surface 21a and the end portion of the distal end side guide portion 23 including the distal end side guide surface 23a are inserted between the projecting portions 10b of the anvil roller 10. Because of this, the anvil roller 10 positioned in the non-contact ranges E2, E4 and the lift cam 18 are engaged with each other such that movement of the anvil roller 10 in a direction (the left-right direction in FIG. 7) orthogonal to the moving direction of the anvil roller 10 and the direction of energization by the energizing member 25 is regulated.

The operation of the anvil roller 10 by the lift cam 18 that configures part of the displacement mechanism 100 will now be described below.

In the process in which the anvil roller 10 of FIG. 6 moves in the contact range E3, the welding surfaces 10d of the anvil roller 10 are pressed by the energizing force produced by the energizing member 25 (FIG. 3) against the output surfaces 6e of the ultrasonic horn 6 with the workpiece W sandwiched between them.

In this pressed state, ultrasonic vibrations are applied by the ultrasonic horn 6, whereby the workpiece W is ultrasonically welded.

When the anvil roller 10 of FIG. 6 starts moving from the contact range E3 toward the non-contact ranges E2, E4, the supported surface 10c of the anvil roller 10 contacts the sloping surfaces of the guide surfaces 21a, 23a. Moreover, when the movement of the anvil roller 10 progresses, the anvil roller 10 rollingly contacts the guide surfaces 21a, 23a (the sloping surfaces) and moves along the sloping surfaces and gradually away from the ultrasonic horn 6 counter to the energizing force produced by the energizing member 25 (FIG. 3). Additionally, when the movement of the anvil roller 10 further progresses, the anvil roller 10 becomes supported by the flat surfaces of the guide surfaces 21a, 23a. In other words, both guide surfaces 21a, 23a have a shape that can guide the anvil roller 10 in directions in which the output surfaces 6e and the welding surfaces 10d move away from each other in directions normal to these surfaces counter to the energizing force of the energizing member 25.

In this supported state, the welding surfaces 10d of the anvil roller 10 are a preset dimension (a dimension larger than the amplitude of the ultrasonic vibrations) away from the output surfaces 6e of the ultrasonic horn 6. For that reason, wear and deterioration of the ultrasonic horn 6 and the anvil roller 10 caused by the ultrasonic vibrations applied by the ultrasonic horn 6 being transmitted to the anvil roller 10 can be prevented.

Conversely, when the anvil roller 10 starts moving from the non-contact ranges E2, E4 toward the contact range E3, the anvil roller 10 rollingly contacts the guide surfaces 21a, 23a and moves along the sloping surfaces and gradually toward the ultrasonic horn 6 due to the energizing force produced by the energizing member 25 (FIG. 3). Moreover, when the movement of the anvil roller 10 progresses, the anvil roller 10 reaches the contact range E3.

In other words, the energizing mechanism (the energizing member 25, the retention lever 19, and the pivot shaft 19b) and the lift cam 18 correspond to the displacement mechanism 100 that displaces the anvil roller 10 relative to the ultrasonic horn 6 such that the output surfaces 6e and the welding surfaces 10d move toward each other in the contact range E3 and such that the output surfaces 6e and the welding surfaces 10d move away from each other in the non-contact ranges E2, E4.

As described above, the anvil roller 10 is energized toward the ultrasonic horn 6, and the outer surface of the anvil roller 10 positioned in the non-contact ranges E2, E4 is pressed by this energizing force against the guide surfaces 21a, 23a of the lift cam 18.

Additionally, both guide surfaces 21a, 23a can guide the anvil roller 10 in directions in which the output surfaces 6e and the welding surfaces 10d move away from each other as the anvil roller 10 moves from the contact range E3 to the non-contact ranges E2, E4.

For that reason, in a state in which the anvil roller 10 has moved to the contact range E3, the output surfaces 6e and the welding surfaces 10d can be moved toward each other in directions normal to these surfaces by the energizing force of the energizing member 25 (FIG. 3) to ultrasonically weld the workpiece W between them. Conversely, the output surfaces 6e and the welding surfaces 10d can be moved away from each other as a result of the anvil roller 10 being guided along the guide surfaces 21a, 23a as the anvil roller 10 moves from the contact range E3 to the non-contact ranges E2, E4.

Next, details about the ultrasonic welding apparatus 1 will be described. The ultrasonic welding apparatus 1 forms the weld portions S in the workpiece W such that the welding region α in which the workpiece W of FIG. 1(c) is welded extends in the first direction L1 and the non-welding region Δ that extends on one side of the welding region α and in which it is not necessary to weld the workpiece W is consecutive with the welding region α in the first direction L1. In the case of the present example, the first direction L1 is the width direction orthogonal to the waist direction of the wearable article 20.

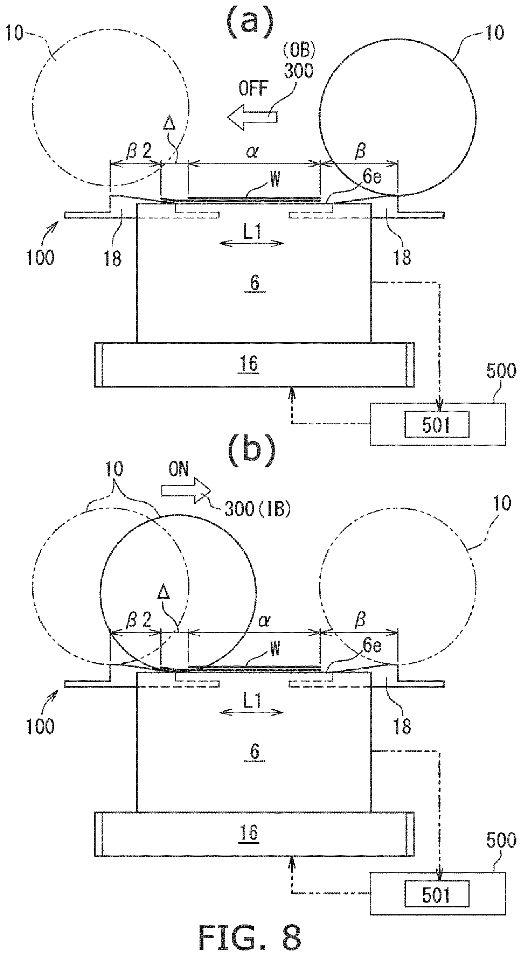

The ultrasonic welding apparatus 1 of FIG. 8(a) includes an ultrasonic generator 16 and a control device 500. The ultrasonic generator 16 causes the horn 6 to vibrate to apply ultrasonic energy to the workpiece W sandwiched between the anvil 10 and the horn 6.

The moving mechanism 300, as in FIG. 8(a) and FIG. 8(b), moves the anvil 10 in the first direction L1 such that the horn 6 abuts against the anvil 10 via the workpiece W in the welding region α and the non-welding region Δ and reciprocally moves the anvil 10 between the first overrun region β, where the anvil 10 exceeds the other side of the welding region α, and the non-welding region Δ and second overrun region β2.

The displacement mechanism 100 includes the lift cam 18 that guides the anvil 10 such that the anvil 10 reaches a first level and the aforementioned energizing member 25 (FIG. 3) that energizes the anvil 10 such that the anvil 10 touches the horn 6.

The lift cam 18 of the displacement mechanism 100 guides the anvil 10 such that the anvil 10 reaches a distal first level away from a surface level of the horn 6 so that the anvil 10 does not abut against the horn 6 at end portions (stroke ends of the anvil) of the non-welding region Δ and the second overrun region β2 far from the welding region α and allows the anvil 10 to move to a proximal second level where the anvil 10 touches the horn 6 in the welding region α. It will be noted that the lift cam 18 may be divided into two in the first direction L1.

On an outbound path OB of FIG. 8(a), the control device 500 performs control such that in the first overrun region β the anvil 10 becomes displaced from the first level to the second level and the anvil 10 abuts against the horn 6 not via the workpiece W in a non-driven state (including a weakly driven state) in which the horn 6 is not being caused to generate ultrasonic energy.

On an inbound path IB of FIG. 8(b), the control device 500 performs control such that in the non-welding region Δ the anvil 10 becomes displaced from the first level to the second level and the anvil 10 abuts against the horn 6 via the workpiece W in a driven state in which the horn 6 is being caused to generate ultrasonic energy.

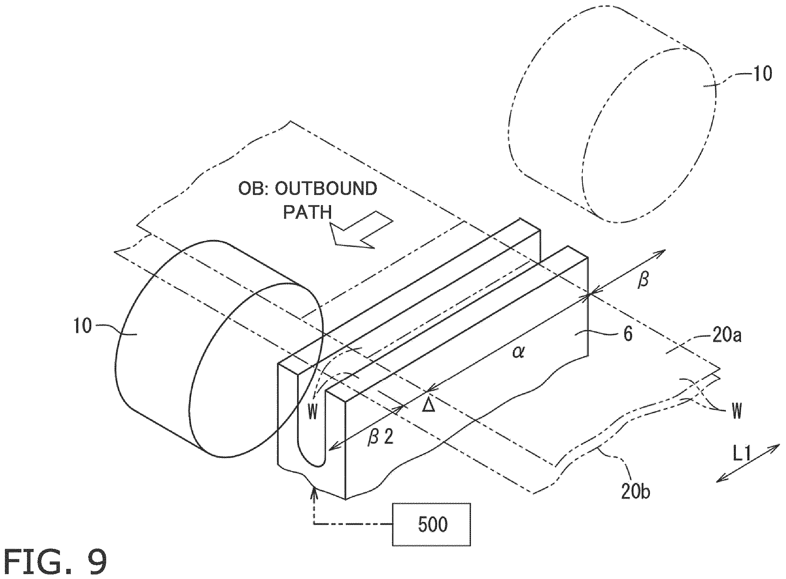

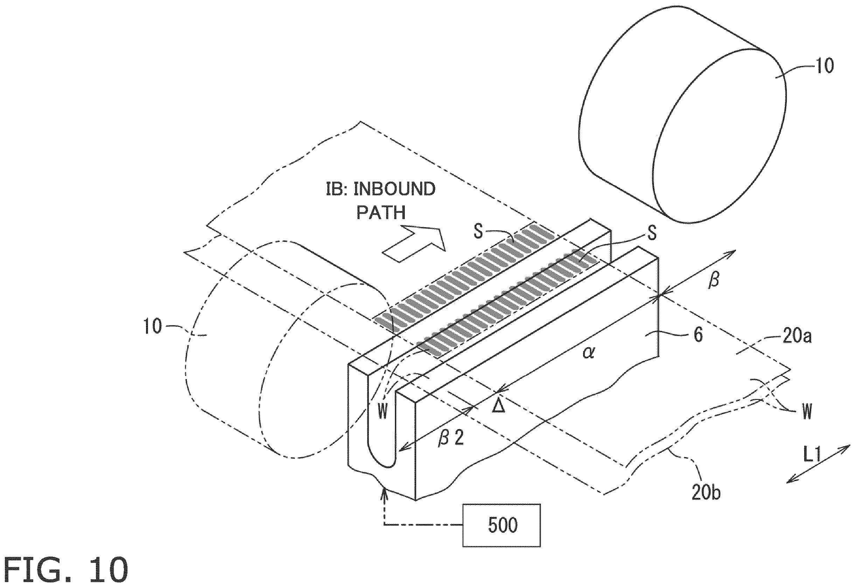

The control device 500 performs control to apply ultrasonic energy to the workpiece W only on, out of the outbound path OB on which the anvil 10 of FIG. 9 proceeds from the other side in the first direction L1 to the one side and the inbound path IB on which the anvil 10 proceeds from the one side of FIG. 10 to the other side, the inbound path IB.

As shown in FIG. 8(b), the welding drum 3 conveys the workpiece W such that the anvil 10 on the inbound path IB becomes displaced from the first level to the second level in the non-welding region Δ and the anvil 10 abuts against the horn 6 via the workpiece W in the non-welding region Δ. That is, the workpiece W is disposed eccentrically on the second overrun region β2 side by an amount corresponding to the non-welding region Δ relative to the center of the horn 6.

For that reason, on the inbound path IB, the anvil (roller) 10 impacts on (abuts against) the driven horn 6 from the second overrun region β2 via the non-welding region Δ of the workpiece W and does not directly impact on the horn 6.

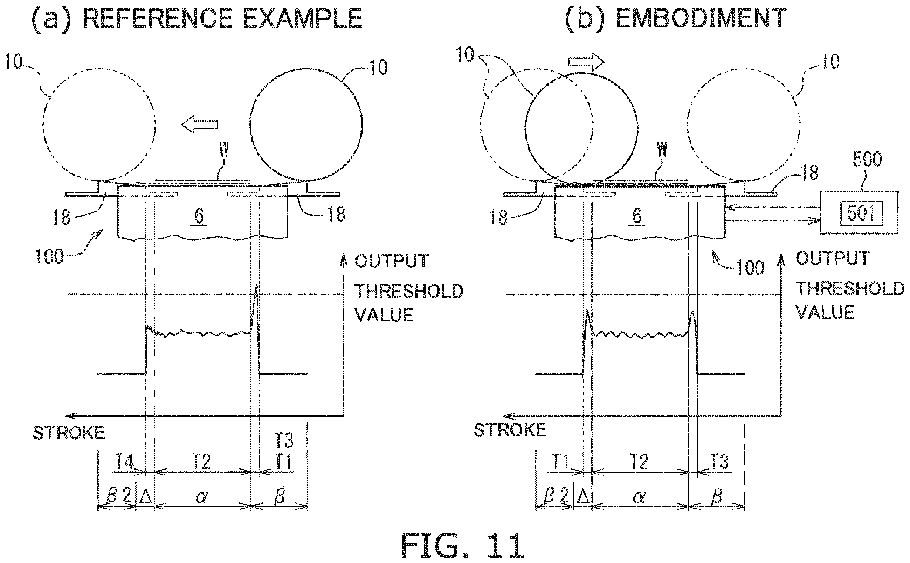

As shown in FIG. 11(b), the control device 500 has an output unit 501 to which the sonic output of the horn 6 is input and which outputs an error when the sonic output reaches a predetermined threshold value.

Next, the operation of the ultrasonic welding apparatus 1, including a method of forming the weld portions S of FIG. 10, will be described.

In the present example, as shown in FIG. 1(c), the weld portions S are formed in both ends of the waist portion of the wearable article 20. As shown in FIG. 1(a), the weld portions S are formed in the vicinity of a cutting line between adjacent wearable articles 20. As shown in FIG. 10, the weld portions S are intermittently formed in the first direction L1 in the welding region α.

In the present welding method, the control device 500 performs control to apply ultrasonic energy to the workpiece W only on the inbound path IB out of the outbound path OB of FIG. 9 on which the anvil 10 proceeds from the other side in the first direction L1 to the one side and the inbound path IB of FIG. 10 on which the anvil 10 proceeds from the one side to the other side.

First, the outbound path OB of FIG. 8(a) will be described. In this drawing, the anvil 10 is retained at the distal first level by the lift cam 18 in a starting position indicated by the solid line. As the anvil 10 proceeds in the first direction L1 from the starting position (the first overrun region β), it becomes displaced along the sloping surface of the lift cam 18 from the first level to the proximal second level and abuts against the surfaces (the output surfaces 6e) of the horn 6.

After this abutment, the anvil 10 crosses over the welding region α and the non-welding region Δ of the workpiece W, ascends along the sloping surface of the lift cam 18 to the first level again, and proceeds to a stroke end (the second overrun region β2) indicated by the virtual line.

On the outbound path OB described above, the horn 6 substantially does not vibrate. Consequently, as shown in FIG. 9, on the outbound path OB the workpiece W is not welded.

Next, the inbound path IB of FIG. 10 will be described. On the inbound path IB, the anvil 10 returns from the aforementioned stroke end (the second overrun region β2) indicated by the virtual line to the starting position (the first overrun region β) indicated by the solid line. As shown in FIG. 8(b), at the aforementioned stroke end (the second overrun region β2), the anvil 10 is retained at the first level, where it does not touch the horn 6, by the lift cam 18.

On the inbound path IB of FIG. 8(b), as the anvil 10 proceeds from the stroke end to the opposite side in the first direction L1, it becomes displaced along the sloping surface of the lift cam 18 from the distal first level to the proximal second level and abuts against the surfaces (the output surfaces 6e) of the horn 6 via the workpiece W in the non-welding region Δ. On the inbound path IB the horn 6 intermittently repeats ultrasonic welding. However, since the thickness of the workpiece W is thin in the non-welding region Δ, welding energy is not generated in the workpiece W.

At the time of the abutment on the inbound path IB, the horn output shown in FIG. 11(b) is larger than it is during welding because of added energy caused by the impact. However, since the anvil 10 does not directly abut against (metal-on-metal contact) the horn 6 but abuts against the horn 6 via the non-welding region Δ of the workpiece W, the impact energy does not increase much, and for that reason the horn output is smaller than the predetermined threshold value. For that reason, there is no concern that the control device 500 will output an error.

In FIG. 11(b), the anvil 10 indicated by the solid line passes through the welding region α after passing through the non-welding region Δ. At this time, the horn 6 ultrasonically vibrates and applies ultrasonic energy to the workpiece W between itself and the anvil 10 while sandwiching the welding region α of the workpiece W between itself and the anvil 10. Because of this, numerous weld portions S are formed in two rows in the welding region α as indicated by the gray coloring in FIG. 10.

As shown in FIG. 11(b), during this welding, the output of the horn resulting from the ultrasonic vibrations appears as a waveform. The level of this output is lower than the aforementioned output level in the non-welding region Δ.

In FIG. 11(b), the anvil 10 that has passed through the aforementioned welding region α heads toward the starting position indicated by the virtual line. At this time, the anvil 10 passes through the first overrun region β where the workpiece W is not disposed on the surface of the horn 6. In this first overrun region β, the horn 6 directly impacts on (metal-on-metal contact) the anvil 10 while repeatedly ultrasonically vibrating. For that reason, the horn output is larger than it is during the aforementioned welding.

However, the horn output is set so as to be smaller than the predetermined threshold value. Consequently, there is no concern that an error output will be output.

Thereafter, the anvil 10 ascends along the sloping surface of the lift cam 18 to the first level again and returns to the starting position indicated by the virtual line.

In this way, in the present example of FIG. 11(b), a falling impact time T1 and a metal-on-metal contact time T3 when shock is large are configured to occur separately from each other before and after a welding time T2, so the falling impact time T1 and the metal-on-metal contact time T3 when shock is large do not coincide. For that reason, the horn output can be prevented from significantly increasing, and the occurrence of an error output can be prevented.

To facilitate understanding of such advantageous effects of the present example, a case where the falling impact time T1 and the metal-on-metal contact time T3 appear simultaneously will be described using FIG. 11(a).

FIG. 11(a) shows a reference example that is not included in the present invention. As shown in this drawing, when the anvil 10 that is at the first level indicated by the solid line descends on the sloping surface of the lift cam 18 and directly abuts against (metal-on-metal contact) the horn 6, shock caused by the falling impact of the anvil 10 coincides with shock caused by metal-on-metal contact. Moreover, the horn 6 is ultrasonically vibrating, and the energy of the impact with the anvil 10 caused by this vibration coincides with it. For that reason, as shown in this drawing, the horn output becomes significantly larger than it is during the welding time T2 and tends to exceed the predetermined threshold value. It will be noted that T4 is a non-welding time, but because the workpiece W is thin, the horn output becomes larger than it is during the welding time T2.

In contrast, in the present example of FIG. 11(b), the horn output can be prevented from significantly increasing, and the occurrence of an error output can be prevented.

The specific embodiment described above includes an ultrasonic welding apparatus invention having the following configurations.

The apparatus of the present invention is an ultrasonic welding apparatus 1 that forms weld portions S in a workpiece W such that a welding region α in which the workpiece W is welded extends in a first direction L1 and a non-welding region Δ that extends on one side of the welding region α and in which it is not necessary to weld the workpiece W is consecutive with the welding region α in the first direction L1, the ultrasonic welding apparatus 1 including:
a horn 6 and an anvil 10;
an ultrasonic generator 16 that causes the horn 6 to vibrate to apply ultrasonic energy to the workpiece W sandwiched between the anvil 10 and the horn 6;
a moving mechanism 300 that moves the anvil 10 in the first direction L1 such that the horn 6 abuts against the anvil 10 via the workpiece W in the welding region α and the non-welding region Δ and reciprocally moves the anvil 10 between an overrun region β, where the anvil 10 overruns the other side of the welding region α, and the non-welding region Δ;
a displacement mechanism 100 that guides the anvil 10 such that the anvil 10 reaches a first level away from a surface level of the horn 6 so that the anvil 10 does not abut against the horn 6 at end portions (stroke ends of the anvil) of the non-welding region Δ and the overrun region β far from the welding region α and allows the anvil 10 to move to a second level where the anvil 10 touches the horn 6 in the welding region α; and
a control device 500 that performs control such that in the overrun region β the anvil 10 becomes displaced from the first level to the second level and the anvil 10 abuts against the horn 6 not via the workpiece W in a non-driven state (including a weakly driven state) in which the horn 6 is not being caused to generate ultrasonic energy.

According to the apparatus of the present invention, in the overrun region β the anvil 10 is displaced from the first level to the second level relative to the non-driven horn and the anvil 10 abuts against the horn 6 not via the workpiece W. Consequently, although at this time shock caused by the abutment occurs, energy caused by ultrasonic vibration is not applied. As a result, excessive shocks can be inhibited, and deterioration of the horn can be inhibited.

Preferably, the control device 500 performs control such that in the non-welding region Δ the anvil 10 becomes displaced from the first level to the second level and the anvil 10 abuts against the horn 6 via the workpiece W in a driven state in which the horn 6 is being caused to generate ultrasonic energy.

In this case, although in the non-welding region Δ shocks caused by vibration occur in the horn 6 since the horn 6 is in a driven state, metal-on-metal contact can be prevented because the horn 6 abuts against the anvil 10 via the non-welding region Δ of the workpiece W. Consequently, the shocks that occur in the horn become smaller.

In a preferred example, the displacement mechanism 100 may include a lift cam 18 that guides the anvil 10 such that the anvil 10 reaches the first level and an energizing member 25 that energizes the anvil 10 such that the anvil 10 touches the horn 6.

In a preferred example, the ultrasonic welding apparatus 1 may include a welding drum 3 that conveys the workpiece W along its outer peripheral surface while rotating and in which the horn 6 and the anvil 10 is disposed oppose to each other inward and outward in its radial direction.

Preferably still, the workpiece W includes a front abdominal portion 20a and a back portion 20b of a wearable article 20, and the back portion 20b covers the front abdominal portion 20a and has the non-welding region Δ that protrudes on the one side in the first direction L1 from the front abdominal portion 20a, and
the control device 500 performs control to apply ultrasonic energy to the workpiece W only on, out of an outbound path OB on which the anvil 10 proceeds from the other side to the one side in the first direction L1 and an inbound path IB on which the anvil 10 proceeds from the one side to the other side, the inbound path IB.

In this case, ultrasonic welding of the workpiece W is performed only on the inbound path IB in the reciprocating movement. Consequently, welding strength is stable and the welding marks whose outward appearance is excellent are obtained.

Preferably still, the welding drum 3 conveys the workpiece W such that the anvil 10 becomes displaced from the first level to the second level in the non-welding region Δ and the anvil 10 abuts against the horn 6 via the workpiece W in the non-welding region Δ.

Because the welding drum 3 conveys the workpiece W in this way, when the anvil 10 is displaced from the first level to the second level the anvil 10 abuts against the horn 6 via the non-welding region Δ of the workpiece W, and the shock of the impact caused by falling is mitigated by the non-welding region Δ of the workpiece W.

Preferably still, the control device 500 has an output unit 501 to which the sonic output of the horn 6 is input and which outputs an error when the sonic output reaches a predetermined threshold value.

In this case, when the sonic output becomes excessive, the control device 500 and/or an operator can know this, and deterioration of the horn can be inhibited.

In addition, the aforementioned specific embodiment includes an ultrasonic welding method invention having the following processes.

The method invention of the present invention is an ultrasonic welding method that forms weld portions S in a workpiece W such that a welding region α in which the workpiece W is welded extends in a first direction L1 and a non-welding region Δ that extends on one side of the welding region α and in which it is not necessary to weld the workpiece W is consecutive with the welding region α in the first direction L1, the ultrasonic welding method including:
causing a horn 6 to vibrate to apply ultrasonic energy to the workpiece W sandwiched between an anvil 10 and the horn 6;
moving the anvil 10 in the first direction L1 such that the horn 6 abuts against the anvil 10 via the workpiece W in the welding region α and the non-welding region Δ and reciprocally moving the anvil 10 between an overrun region β, where the anvil 10 overruns the other side of the welding region α, and the non-welding region Δ;
guiding the anvil 10 such that the anvil 10 reaches a first level away from a surface level of the horn 6 so that the anvil 10 does not abut against the horn 6 at end portions of the non-welding region Δ and the overrun region β far from the welding region α and causing the anvil 10 to move to a second level where the anvil 10 touches the horn 6 in the welding region α; and
performing control such that in the overrun region β the anvil 10 becomes displaced from the first level to the second level and the anvil 10 abuts against the horn 6 not via the workpiece W in a non-driven state in which the horn 6 is not being caused to generate ultrasonic energy.

According to the method of the present invention, in the overrun region β the anvil 10 is displaced from the first level to the second level relative to the non-driven horn and the anvil 10 abuts against the horn 6 not via the workpiece W. Consequently, although at this time shock caused by the abutment occurs, energy caused by ultrasonic vibration is not applied. As a result, excessive shocks can be inhibited, and deterioration of the horn can be inhibited.

Preferably, performing control such that in the non-welding region Δ the anvil 10 becomes displaced from the first level to the second level and the anvil 10 abuts against the horn 6 via the workpiece W in a driven state in which the horn 6 is being caused to generate ultrasonic energy.

In this case, although in the non-welding region Δ shocks caused by vibration occur in the horn 6 since the horn 6 is in a driven state, metal-on-metal contact can be prevented because the horn 6 abuts against the anvil 10 via the non-welding region Δ of the workpiece W. Consequently, the shocks that occur in the horn become smaller.

Preferably still, the workpiece W includes a front abdominal portion 20a and a back portion 20b of a wearable article 20, and the back portion 20b covers the front abdominal portion 20a and has the non-welding region Δ that protrudes on the one side in the first direction L1 from the front abdominal portion 20a, and
the control device 500 performs control to apply ultrasonic energy to the workpiece W only on, out of an outbound path OB on which the anvil 10 proceeds from the other side to the one side in the first direction L1 and an inbound path IB on which the anvil 10 proceeds from the one side to the other side, the inbound path IB.

In this case, ultrasonic welding of the workpiece W is performed only on the inbound path IB in the reciprocating movement. Consequently, welding strength is stable and welding marks whose outward appearance is excellent are obtained.

Preferably still, the ultrasonic welding method includes inputting the sonic output of the horn 6 and outputting an error when the sonic output reaches a predetermined threshold value.

Because the welding drum 3 conveys the workpiece W in this way, when the anvil 10 is displaced from the first level to the second level, the anvil 10 abuts against the horn 6 via the non-welding region Δ of the workpiece W, and the shock of the impact caused by falling is mitigated by the non-welding region Δ of the workpiece W.

The features described and/or illustrated in association with each of the aforementioned embodiments can be used in the same or similar form in one or more other embodiments and/or in combination with, or instead of, features of the other embodiments.

While preferred embodiments have been described above with reference to the drawings, a variety of obvious changes and modifications will readily occur to those skilled in the art upon reading the present specification.

For example, the anvil may be disposed on the radial direction inner side of the outer peripheral surface of a web support member, and the horn may be disposed on the outer side of the same outer peripheral surface.

Furthermore, as the moving mechanism, a structure such as that of US 2017/0027762 A1 may be employed.

Furthermore, the drum may be provided with just one set of the horn and the anvil or may be provided with plural sets.

Furthermore, parts other than what are called side seals of a pants type wearable article may be welded.

Consequently, such changes and modifications are to be interpreted as being within the scope of the present invention as defined by the claims.

### INDUSTRIAL APPLICABILITY

The present invention can be applied to equipment for producing disposable wearable articles such as disposable pants, diapers, and sanitary napkins and can also be applied to equipment for producing medical wound dressings.

### REFERENCE SIGNS LIST

1: Ultrasonic Welding Apparatus, 2: Infeed Roller, 3: Welding Drum, 4: Outfeed Roller 5: Sheet Retention Drum (Retention Member), 5a: Recessed Groove
6: (Ultrasonic) Horn, 6a: Input-side End Portion, 6b: Output-side End Portion, 6c: Input Surface, 6d: Slit, 6e: Output Surfaces
7: Anvil Unit, 8: Anvil Retention Drum, 9: Cam Drum, 9a: Cam Groove 10: Anvil (Roller), 10a: Roller Body, 10b: Projecting Portions, 10c: Supported Surface, 10d: Welding Surfaces
11: Retention Member, 12: Retention Member Body
14: Cam Protrusion, 15: Engagement Protrusions
16: Ultrasonic Generator, 16a: Slit
17: Rails, 17a: Engagement Grooves
18: Lift Cam, 19: Retention Lever, 19a: Rotating Shaft, 19b: Pivot Shaft
20: Wearable Article (Disposable Diaper), 20a: Front Abdominal Portion, 20b: Back Portion,
20c: Crotch Portion
200: Absorber
21: Base End Side Guide Portion, 21a: Base End Side Guide Surface
22: Central Portion, 22a: Non-guide Surface
23: Distal End Side Guide Portion, 23a: Distal End Side Guide Surface
25: Energizing Member, 500: Control Device, 501: Output Unit
100: Displacement Mechanism, 300: Moving Mechanism
E1: Range, E2, E4: Non-contact Ranges, E3: Contact Range
F: Elastic Members, S: Weld Portions, S1 (S2): First (Second) Stretchable Sheet
IB: Inbound Path, OB: Outbound Path, L1: First Direction
T1: Falling Impact Time, T2: Welding Time, T3: Metal-on-metal Contact Time
α: Welding Region, β: Overrun Region
Δ: Non-welding Region

## Claims

1. An ultrasonic welding apparatus 1 that forms a weld portion S in a workpiece W such that a welding region α in which the workpiece W is welded extends in a first direction L1 and a non-welding region Δ that extends on one side of the welding region α and in which it is not necessary to weld the workpiece W is consecutive with the welding region α in the first direction L1, the ultrasonic welding apparatus 1 comprising:
a horn 6 and an anvil 10;
an ultrasonic generator 16 configured to cause the horn 6 to vibrate to apply ultrasonic energy to the workpiece W sandwiched between the anvil 10 and the horn 6;
a moving mechanism 300 configured to move the anvil 10 in the first direction L1 such that the horn 6 abuts against the anvil 10 via the workpiece W in the welding region α and the non-welding region Δ and reciprocally move the anvil 10 between an overrun region β, where the anvil 10 exceeds an other side of the welding region α, and the non-welding region Δ;
a displacement mechanism 100 configured to guide the anvil 10 such that the anvil 10 reaches a first level away from a surface level of the horn 6 so that the anvil 10 does not abut against the horn 6 at end portions of the non-welding region Δ and the overrun region β far from the welding region α and allow the anvil 10 to move to a second level where the anvil 10 touches the horn 6 in the welding region α; and
a control device 500 configured to perform control such that in the overrun region β the anvil 10 becomes displaced from the first level to the second level and the anvil 10 abuts against the horn 6 not via the workpiece W in a non-driven state in which the horn 6 is not being caused to generate ultrasonic energy.

2. The ultrasonic welding apparatus 1 according to claim 1, wherein
the control device 500 is configured to perform control such that in the non-welding region Δ the anvil 10 becomes displaced from the first level to the second level and the anvil 10 abuts against the horn 6 via the workpiece W in a driven state in which the horn 6 is being caused to generate ultrasonic energy.

3. The ultrasonic welding apparatus 1 according to claim 1 or 2, wherein
the displacement mechanism 100 includes a lift cam 18 configured to guide the anvil 10 such that the anvil 10 reaches the first level and an energizing member 25 configured to energize the anvil 10 such that the anvil 10 touches the horn 6.

4. The ultrasonic welding apparatus 1 according to claim 3 comprising
a welding drum 3 configured to convey the workpiece W along its outer peripheral surface while rotating and in which the horn 6 and the anvil 10 is disposed oppose to each other inward and outward in its radial direction.

5. The ultrasonic welding apparatus 1 according to claim 4, wherein
the workpiece W includes a front abdominal portion 20a and a back portion 20b of a wearable article 20, and the back portion 20b covers the front abdominal portion 20a and has the non-welding region Δ that protrudes on the one side in the first direction L1 from the front abdominal portion 20a, and
the control device 500 is configured to perform control to apply ultrasonic energy to the workpiece W only on, out of an outbound path OB on which the anvil 10 proceeds from an other side to the one side in the first direction L1 and an inbound path IB on which the anvil 10 proceeds from the one side to the other side, the inbound path IB.

6. The ultrasonic welding apparatus 1 according to claim 5, wherein
the welding drum 3 is configured to convey the workpiece W such that the anvil 10 becomes displaced from the first level to the second level in the non-welding region Δ and the anvil 10 abuts against the horn 6 via the workpiece W in the non-welding region Δ.

7. The ultrasonic welding apparatus 1 according to claim 6, wherein
the control device 500 has an output unit 501 to which a sonic output of the horn 6 is configured to be input and which is configured to output an error when the sonic output reaches a predetermined threshold value.

8. An ultrasonic welding method that forms a weld portion S in a workpiece W such that a welding region α in which the workpiece W is welded extends in a first direction L1 and a non-welding region Δ that extends on one side of the welding region α and in which it is not necessary to weld the workpiece W is consecutive with the welding region α in the first direction L1, the ultrasonic welding method comprising:
causing a horn 6 to vibrate to apply ultrasonic energy to the workpiece W sandwiched between an anvil 10 and the horn 6;
moving the anvil 10 in the first direction L1 such that the horn 6 abuts against the anvil 10 via the workpiece W in the welding region α and the non-welding region Δ and reciprocally moving the anvil 10 between an overrun region β, where the anvil 10 exceeds an other side of the welding region α, and the non-welding region Δ;
guiding the anvil 10 such that the anvil 10 reaches a first level away from a surface level of the horn 6 so that the anvil 10 does not abut against the horn 6 at end portions of the non-welding region Δ and the overrun region β far from the welding region α and causing the anvil 10 to move to a second level where the anvil 10 touches the horn 6 in the welding region α; and
performing control such that in the overrun region β the anvil 10 becomes displaced from the first level to the second level and the anvil 10 abuts against the horn 6 not via the workpiece W in a non-driven state in which the horn 6 is not being caused to generate ultrasonic energy.

9. The ultrasonic welding method according to claim 8, wherein
performing control such that in the non-welding region Δ the anvil 10 becomes displaced from the first level to the second level and the anvil 10 abuts against the horn 6 via the workpiece W in a driven state in which the horn 6 is being caused to generate ultrasonic energy.

10. The ultrasonic welding method according to claim 9, wherein
the workpiece W includes a front abdominal portion 20a and a back portion 20b of a wearable article 20, and the back portion 20b covers the front abdominal portion 20a and has the non-welding region Δ that protrudes on the one side in the first direction L1 from the front abdominal portion 20a, and
the control device 500 performs control to apply ultrasonic energy to the workpiece W only on, out of an outbound path OB on which the anvil 10 proceeds from an other side to the one side in the first direction L1 and an inbound path IB on which the anvil 10 proceeds from the one side to the other side, the inbound path IB.

11. The ultrasonic welding method of claim 10 further comprising
inputting sonic output of the horn 6 and outputting an error when the sonic output reaches a predetermined threshold value.
